# EUROPÄISCHE PATENTSCHRIFT

(11) **EP 0 825 192 B1**
(45) Veröffentlichungstag und Bekanntmachung des Hinweises auf die Patenterteilung: **13.11.2002**
(21) Anmeldenummer: 97113457.2
(22) Anmeldetag: 05.08.1997
(51) Int. Cl.: C07D 413/12, C09B 57/04, D06P 1/16

(54) **Benzoxazolylisoindolenine**
Benzoxazolylisoindolenines
Benzoxazolylisoindolénines

(30) Priorität: 16.08.1996 DE 19632921
(43) Veröffentlichungstag der Anmeldung: 25.02.1998
(73) Patentinhaber: DyStar Textilfarben GmbH & Co. Deutschland KG, 65926 Frankfurt am Main (DE)
(72) Erfinder: Lorenz, Manfred, Dr., 51061 Köln (DE)

(56) Entgegenhaltungen:
- EP-A- 0 023 889
- EP-A- 0 684 289
- CH-A- 613 465
- DE-A- 1 670 748

## Beschreibung

Die Erfindung betrifft Benzoxazolylisoindolenine, Verfahren zu ihrer Herstellung sowie ihre Verwendung zum Färben von hydrophoben synthetischen oder halbsynthetischen Materialien.

Aus EP-A 0 684 289 sind Thiazol-isoindolenin-Farbstoffe, aus DE-A 16 70 748, CH 613 465 und EP-A 023 889 Isoindoleninfarbstoffe bekannt, die jedoch noch anwendungstechnische Nachteile aufweisen. Unter anwendungstechnischen Nachteilen sind beispielsweise zu geringe Zieh- oder Aufbauvermögen beim Färben von Polyester oder eine schlechte Lichtechtheit, insbesondere Heißlichtechtheit, wie sie bei Verwendung damit gefärbter Textilien auf dem Automobilsektor gefordert sind, zu verstehen.

Es wurden Benzoxazolylisoindolenine gefunden, die der Formel I oder deren tautomeren Formen entsprechen, 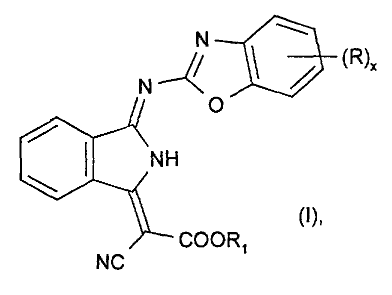 worin
- R₁: für einen gesättigten oder ungesättigten, gegebenenfalls substituierten aliphatischen Rest mit 1 bis 12 Kohlenstoffatomen, der gegebenenfalls durch ein oder mehrere Sauerstoffatome unterbrochen ist, einen gegebenenfalls substituierten cycloaliphatischen Rest mit 5 bis 12 Kohlenstoffatomen oder für einen gegebenenfalls substituierten araliphatischen Rest mit 7 bis 20 Kohlenstoffatomen steht,
- x: eine Zahl von 0 bis 4, vorzugsweise 0, 1 oder 2 bedeutet,
- R: gleich oder verschieden ist und für Halogen, insbesondere Cl oder Br, C₁-C₁₀-Alkyl, insbesondere C₁-C₄-Alkyl, gesättigtes oder ungesättigtes C₁-C₁₀-, insbesondere C₁-C₄-Alkoxy oder -Alkoxyalkoxy, CN, NO₂ oder, sofern x größer als 1, für den Rest eines anellierten Benzorings steht.

Sämtliche in dieser Anmeldung beschriebenen Formeln stellen zwar lediglich eine, sofern mehrere denkbar sind, tautomere Form der jeweiligen Verbindung(en) dar, stehen aber stellvertretend für alle denkbaren tautomeren Formen.

Weiterhin umfaßt ein durch eine Formel beschriebenes E- oder Z-Isomer, insbesondere im Hinblick auf die exocyclische(n) Doppelbindung(en), auch jeweils das andere Isomer. Dies gilt, sofern nicht ausdrücklich etwas anderes gesagt wird.

Als tautomere Formen der Verbindungen der Formel (I) sind beispielsweise die beiden Formeln (Ia) und (Ib) zu nennen.

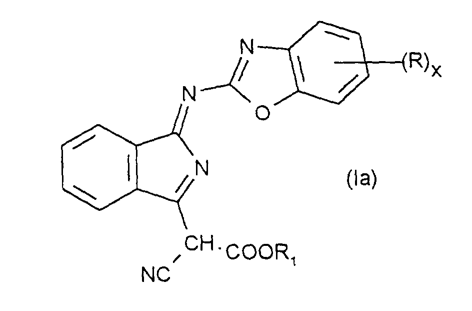

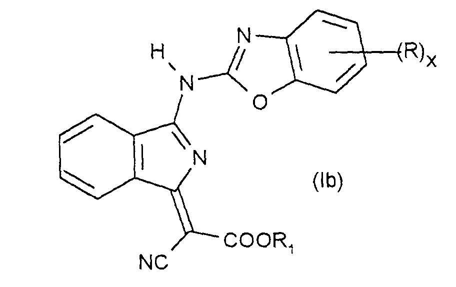

Als mögliche Substituenten für den aliphatischen, cycloaliphatischen sowie araliphatischen Rest in R¹ sind beispielsweise CN, gesättigter oder ungesättigter Oxyrest mit 1 bis 4 C-Atomen, wie C₁-C₄-Alkoxy, Allyloxy und/oder ein Acyloxyrest wie 2-Acetoxyethyl zu nennen.

Geeignete Reste R₁ sind z.B.: Methyl, Ethyl, n-Propyl, Allyl, n-Butyl, n-Pentyl, n-Hexyl, n-Heptyl, n-Octyl, n-Decyl, n-Dodecyl, 2-Methoxy-ethyl, 2-Ethoxy-ethyl, 2-Butoxy-ethyl, 3-Methoxy-propyl, 3-Ethoxy-propyl, 3-Butoxy-propyl, 3-Allyloxypropyl, 2-Ethyl-hexyl, 3-(2-Ethyl-hexyloxy)-propy, Butoxyethoxy-ethyl, 2-Phenoxy-ethyl, Benzyl, Cyclohexylmethyl, Cycloheptyl, Cyclopentyl, Furfuryl. Als verzweigte Reste R₁ kommen vorzugsweise solche mit einer Methylseitenkette in Frage wie z.B.: iso-Butyl oder iso-Pentyl. Bevorzugt sind aliphatische Reste R₁ mit 4 und mehr C-Atomen.

Bevorzugte Reste R sind Chlor, Methyl, Methoxy und Ethoxy.

Besonders bevorzugt sind Farbstoffe der Formel (I), bei denen R₁ für einen geradkettigen aliphatischen Rest mit 4 bis 8 Kohlenstoffatomen steht, der gegebenenfalls durch ein Sauerstoffatom unterbrochen ist, und R für Chlor, Methyl, Methoxy oder Ethoxy steht und x gleich 0 oder 1 bedeutet.

Ganz besonders bevorzugte Farbstoffe der Formel (I) sind solche, bei denen R₁ für einen geradkettigen aliphatischen Rest mit 4 bis 8 Kohlenstoffatomen steht, der noch durch ein Sauerstoffatom unterbrochen sein kann, und x für 0 steht.

Die Erfindung betrifft weiterhin ein Verfahren zur Herstellung von Verbindungen der Formel (I), das dadurch gekennzeichnet ist, daß man entweder eine Verbindung der Formel (II) mit einem Cyanessigsäureester der Formel (III) gemäß der Reaktionsgleichung 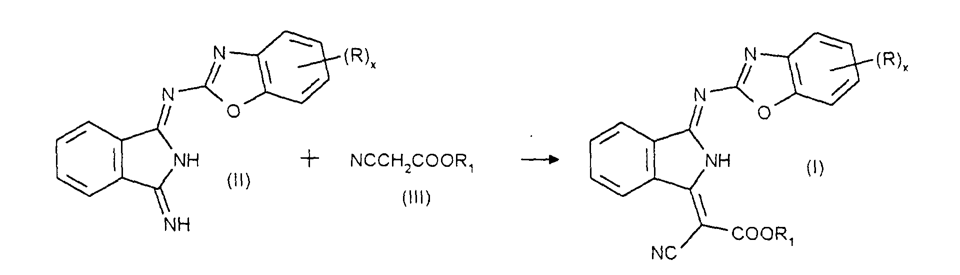 kondensiert
oder eine Verbindung der Formel (IV) mit 2-Amino-benzoxazolen der Formel (V) gemäß der Reaktionsgleichung 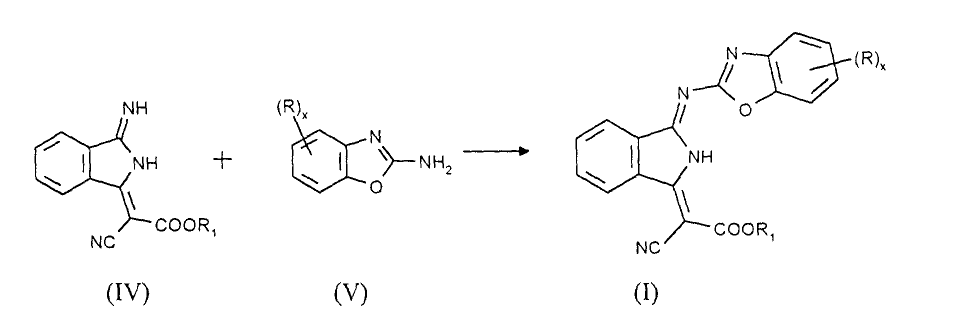 kondensiert,
wobei R, R₁ und x die oben angegebene Bedeutung besitzen.

Bei den Reaktionen wird jeweils ein Äquivalent Ammoniak frei.

Verbindungen der Formel (IV) sind bereits aus DE-A-16 70 748 bekannt.

Besonders bevorzugt erfolgt das erfindungsgemäße Verfahren durch Umsetzung von Verbindungen der Formel (II) mit solchen der Formel (III).

Die Umsetzung von (II) mit (III) bzw. (IV) mit (V), besonders bevorzugt (II) mit (III) erfolgt vorzugsweise in Wasser, in organischen, vorzugsweise polaren organischen Lösungsmitteln oder in Mischungen davon.

Als polare organische Lösungsmittel sind beispielsweise zu nennen: Amide wie Dimethylformamid, Formamid, Dimethylacetamid, N-Methylpyrrolidon, ferner Dimethylsulfoxid, Acetonitril oder Essigsäure. In einer bevorzugten Ausführungsform führt man die Reaktion in einem dem Rest R₁ zugrundeligenden Alkohol durch. Diese Lösungsmittel können alleine oder in Mischung eingesetzt werden.

Das erfindungsgemäße Verfahren erfolgt vorzugsweise bei Reaktionstemperaturen von 50 bis 150°C, insbesondere 60 bis 100°C. Die Komponenten (II) und (III) können in äquivalenten Mengen oder eine von ihnen im Überschuß eingesetzt werden, wobei ein Überschuß an Cyanessigsäureester von 5 bis 50 %, bevorzugt von 5 bis 30 % vorteilhaft ist, da sich überraschenderweise gezeigt hat, daß dies zu besonders kurzen Reaktionszeiten und zu sehr reinen Endprodukten führt.

Bei der Ausgestaltung der vorliegenden Erfindung wurde überraschenderweise gefunden, daß für die Umsetzung von Verbindungen der Formel (II) mit Verbindungen der Formel (III) gemäß dem obigen Reaktionsschema besonders vorteilhaft auch Wasser oder ein wasserhaltiges Medium als Reaktionsmedium verwendet werden kann. Zusätzlich zum Wasser können auch organische Lösungsmittel zugegen sein und zwar bevorzugt solche, die mit Wasser ganz oder teilweise mischbar sind wie z.B. Alkohole, bevorzugt die Alkohole, die dem Rest R₁ zugrundeliegen, Ketone wie z.B. Aceton, Methylethylketon, Cyclohexanon, Ether wie Tetrahydrofuran und Dioxan, Dimethylformamid, N-Methylpyrrolidon u.a. Es können jedoch auch mit Wasser nicht mischbare Lösungsmittel zugesetzt werden, um z.B. die Kristallinität zu verbessern und besondere Kristallformen zu erzielen. Diese Lösungsmittel können von Anfang an zugegen sein oder aber auch erst im Verlauf der Reaktion zugegeben werden.

In einer besonders bevorzugten Ausführungsform des erfindungsgemäßen Verfahrens wird in Gegenwart einer organischen Säure gearbeitet. Diese führt in der Regel zu einer Beschleunigung der Reaktion, wobei oft auch höhere Ausbeuten und eine höhere Reinheit erzielt werden. Geeignete organische Säuren sind z.B. niedere aliphatische, gesättigte oder ungesättigte Mono- oder Dicarbonsäuren wie z.B. Ameisensäure, Essigsäure, Propionsäure, Buttersäure, Oxalsäure, Fumarsäure, Maleinsäure, Bernsteinsäure, Glutarsäure, Adipinsäure, aber auch aromatische Säuren wie beispielsweise Benzoesäure, Phthalsäure, Phenylessigsäure, Iso- und Terephthalsäure. Die Säuren werden bevorzugt in Mengen von 0,2 bis 3 Äquivalenten, vorzugsweise 1 bis 2 Äquivalenten, bezogen auf die Komponenten II, III, IV oder V, zugegeben. Aber auch höhere Mengen an Säure können eingesetzt werden, bevorzugt dann, wenn die Säure gleichzeitig als Lösungsmittel dient, beispielsweise Essigsäure.

Verwendet man Wasser oder ein überwiegend wäßriges Medium als Reaktionsmedium, so ist es vorteilhaft oberflächenaktive Substanzen wie Tenside, Dispergiermittel, Emulgatoren und Netzmittel zuzugegeben. In Betracht kommen die bekannten nichtionogenen, anionischen und kationischen Hilfsmittel. Solche Verbindungen sind z.B. Salze von Alkylbenzolsulfonsäuren, Alkylphenolsulfonsäuren, Alkylnaphthalinsulfonsäuren, Kondensationsprodukte aus Phenolsulfonsäuren, Fomaldehyd und Harnstoff, Ligninsulfonate, Additionsprodukte von Ethylen- und Propylenoxid an Alkanole, Alkandiole, Phenole, Carbonsäuren, Amine, Carbonsäureamide und ihre Schwefelsäurehalbester, wobei auch Mischungen dieser Verbindungen eingesetzt werden können. Besonders bevorzugt sind jedoch Ligninsulfonate wie z.B. Kraftlignine vom Typ Reax® der Firma Westvaco oder Sulfitlignine vom Typ Ufoxane® der Firma Borregaard.

Die Verbindungen der Formel (IV) werden bevorzugt durch Umsetzung von Aminoiminoisoindolenin der Formel (VI) mit Cyanessigester der Formel (III) 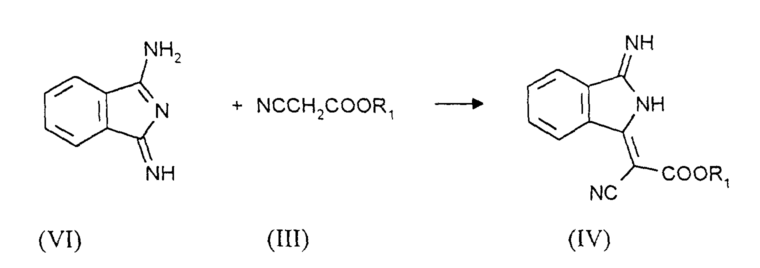 und die Verbindungen der Formel (II) werden bevorzugt durch Umsetzung von Aminoiminoisoindolenin (VI) mit 2-Aminobenzoxazolen der Formel (V) 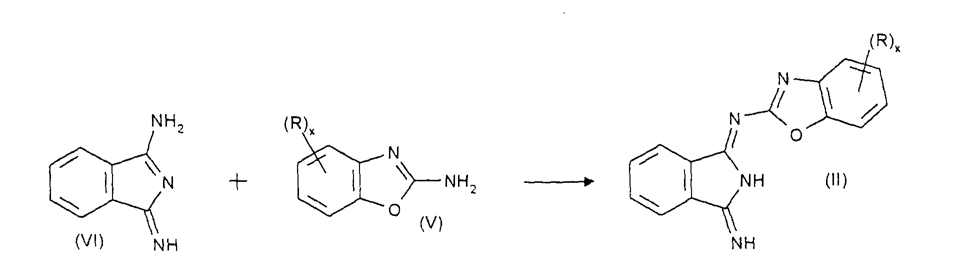 erhalten, wobei R, R₁ und x die obengenannten Bedeutungen besitzen.

Die Herstellung der Monokondensate (IV) bzw. (II) erfolgt vorzugsweise durch Erhitzen der Komponenten VI und III bzw. VI und V in Wasser, einem organischen Lösungsmittel oder Gemischen davon, wobei als Lösungsmittel die bereits obengenannten Lösungsmittel in Frage kommen, insbesondere Amide, z.B. Formamid, Dimethylformamid, Dimethylacetamid und N-Methyl-pyrrolidon, und bevorzugt Alkohole, insbesondere niedere Alkohole wie Methanol, Ethanol, n-Propanol und iso-Propanol, geeignet sind. Auch für die Herstellung der Monokondensate werden vorteilhaft organische Säuren zugegeben, die die Reaktion im allgemeinen beschleunigen. Hierfür kommen die gleichen Säuren in Frage, wie sie bereits vorgehend beschrieben wurden. Ebenso wirkt ein Zusatz von aliphatischen Aminen in der Regel beschleunigend, wobei cycloaliphatische Amine besonders geeignet sind. Solche Amine sind beispielsweise Piperidin, Piperazin oder Morpholin.

Vorteilhaft ist es, die Umsetzung von VI und III in Wasser oder in einem wasserhaltigen Medium, wie es beispielsweise für die Umsetzung von II mit III beschrieben ist, und die Umsetzung von VI und V in organischen Lösungsmittel durchzuführen.

Besonders bevorzugt ist das erfindungsgemäße Verfahren zur Herstellung der Verbindung der Formel (I) dadurch gekennzeichnet, daß die wie oben beschrieben hergestellten Monokondensate (II) bzw. (IV) nicht zwischenisoliert werden und die Reaktion ausgehend von Aminoiminoisoindolenin der Formel (VI) durchgeführt wird.

Die Erfindung betrifft außerdem ein Verfahren zur Herstellung von Verbindungen der Formel (II), das dadurch gekennzeichnet ist, daß man Verbindungen der Formel (VIIa) und/oder (VIIb) mit 2-Amino-benzoxazolen der Formel (V) umsetzt, 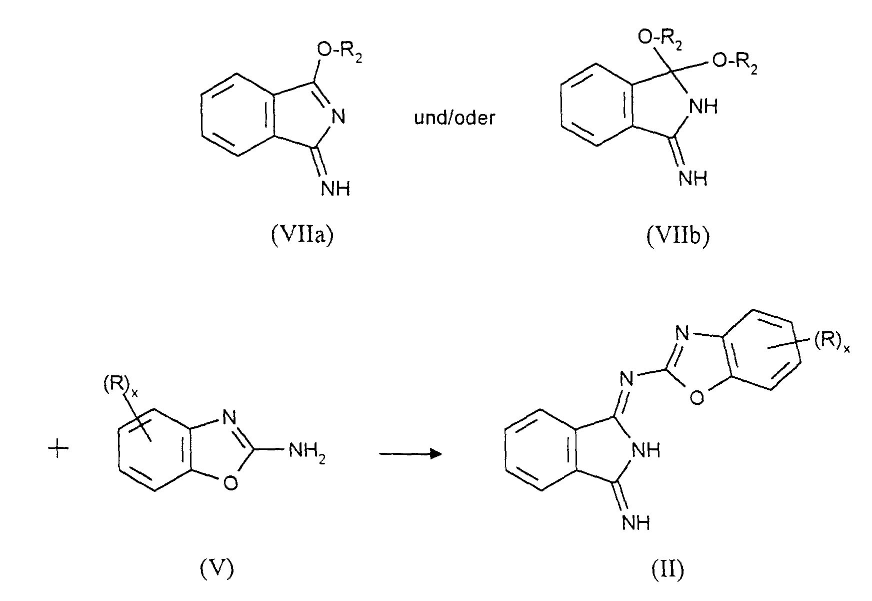 wobei R und x die oben angegebene Bedeutung besitzen und
R₂ für einen aliphatischen Rest, insbesondere für C₁-C₄-Alkyl steht.

Verbindungen der Formel (VIIa) bzw. (VIIb) sind beispielsweise aus DE-A-16 70 748 bekannt. Ihre Herstellung erfolgt vorzugsweise durch Umsetzung von Phthalsäuredinitril mit aliphatischen C₁-C₄-Alkoholen gemäß dem folgenden Reaktionsschema: 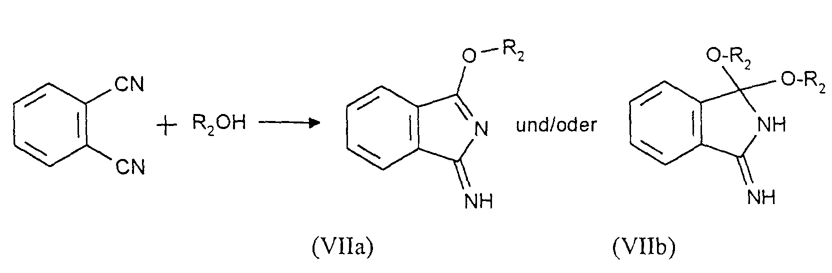 worin R₂ die oben angegebene Bedeutung hat.

Bei den Verbindungen R₂OH handelt es sich insbesondere um Alkohole wie z.B. Methanol, Ethanol, Propanol und Isopropanol, bevorzugt um Methanol. Die Umsetzung von (VIIa) und/oder (VIIb) mit (V) erfolgt vorzugsweise ohne Zwischenisolierung der Verbindungen (VIIa) bzw. (VIIb) unter Zugabe der entsprechenden 2-Amino-benzoxazole (V) und Erhitzen, bis keine Verbindung (VIIa) bzw. (VIIb) mehr nachweisbar ist. Die Reaktionstemperaturen sowohl für die Herstellung von (VIIa) bzw. (VIIb), als auch für (II) liegen bevorzugt zwischen 20 und 80°C.

Die Herstellung von Verbindungen der Formel (VIIa) bzw. (VIIb) erfolgt vorzugsweise in Gegenwart einer Base, vorzugsweise in Gegenwart eines Alkoholats, insbesondere R₂OMe wobei Me ein Alkalimetall wie Na, K oder Li bedeutet und R₂ die oben angegebene Bedeutung besitzt.

Nach beendeter Reaktion wird das als Katalysator dienende Alkoholat vorzugsweise mit einer Säure, beispielsweise mit Essigsäure, neutralisiert und danach das ausgefallene Produkt isoliert oder nach Verdünnen mit einem Lösungsmittel unmittelbar weiter umgesetzt, wobei die bereits genannten Lösungsmittel verwendet werden können.

Die Erfindung betrifft weiterhin Verbindungen der Formel (II) 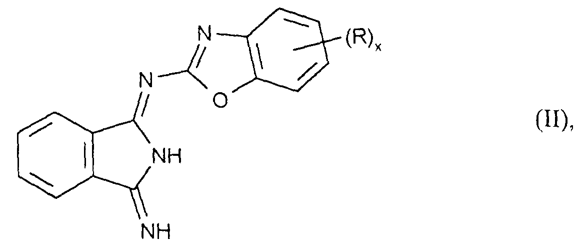 worin
R und x die oben angegebene Bedeutung haben.

Die Erfindung betrifft weiterhin die Verwendung von Farbstoffen der Formeln (I) zum Färben von vollsynthetischen oder hochmolekularen Stoffen. Besonders geeignet sind sie zum Färben oder Bedrucken von synthetischen Fasermaterialien aus aromatischen Polyestern und/oder Celluloseacetaten. Sie besitzen eine hohe Farbstärke und zeigen eine überragende Lichtechtheit, insbesondere eine hohe Heißlichtechtheit, und sind daher besonders zum Färben und Bedrucken von Textilmaterialien für Automobile sowie für das Färben von sogenannten Microfasern geeignet. Sie eignen sich auch für das sogenannte Thermo-Transfer-Printing auf textilen und nichttextilen Substrate z.B. nach dem D2T2 (Dye Diffusion Thermo Transfer) - Prozess für die Bildaufzeichnung. Weiterhin können die Farbstoffe zum Massefärben von Kunststoffen z.B. von Polyethylen, Polypropylen, Styrol, Polycarbonaten sowie von Kunststoffblends wie z.B. ABS verwendet werden. Die Farbstoffe fluoreszieren teilweise und eignen sich daher auch als Fluoreszenzfarbstoffe.

Textilmaterialien aus Polyester können mit den erfindungsgemäßen Farbstoffen nach Art einer Spinnfärbung gefärbt werden bevorzugt jedoch aus wäßriger Suspension. Hierzu werden die Farbstoffe auf allgemein bekannte Weise zu Färbepräparaten verarbeitet, z.B. durch Mahlen in Wasser in Gegenwart von Dispergier- und/oder Füllmitteln. Mit den gegebenenfalls im Vakuum oder durch Zerstäuben getrockneten Präparaten kann man nach Zugabe von Wasser in sogenannter kurzer oder langer Flotte färben, klotzen oder bedrucken.

Zur Herstellung bzw. Verbesserung des Dispersionsgrades kann bei der Mahlung oder der Synthesereaktion ein oberflächenaktives Mittel oder ein Gemisch derartiger Hilfsmittel zugesetzt werden. Selbstverständlich kann die Teilchengröße der Farbstoffpartikel durch eine Mahlbehandlung z.B. Naßperlmahlung, sei es während der Synthese oder im Anschluß daran entsprechend beeinflußt und auf einen geforderten Wert eingestellt werden.

Als Dispergiermittel kommen solche anionischer oder nicht anionischer Natur in Betracht. Neben Dispergiermitteln der einen oder anderen Gruppe können auch Dispergiermittelgemische eingesetzt werden, wobei in erster Linie Gemische von nichtionischen und anionischen Dispergiermitteln gemeint sind, da anionische und kationische Dispergiermittel im Gemisch untereinander zur Bildung von Ausfällungen neigen.

Bei den anionschen Dispergiermitteln haben sich insbesondere Kondensationsprodukte von aromatischen Sulfonsäuren mit Formaldehyd, wie Kondensationsprodukte aus Formaldehyd und Alkylnaphthalinsulfonsäuren oder aus Formaldehyd, Naphthalinsulfonsäuren und Benzolsulfonsäure, Kondensationsprodukte aus gegebenenfalls substituiertem Phenol mit Formaldehyd und Natriumbisulfit als wirksam erwiesen.

Weiterhin kommen vor allem Ligninsulfonate in Betracht, z.B. solche, die nach dem Sulfit- oder Kraft-Verfahren gewonnen werden. Vorzugsweise handelt es sich um Produkte, die z.T. hydrolysiert, oxidiert, propoxyliert oder desulfoniert und nach bekannten Verfahren fraktioniert werden, z.B. nach dem Molekulargewicht oder nach dem Sulfonierungsgrad. Auch Mischungen aus Sulfit- und Kraftligninsulfonaten sind gut wirksam.

Besonders geeignet sind Ligninsulfonate mit einem durchschnittlichen Molekulargewicht zwischen 1.000 und 100.000, einem Gehalt an aktivem Ligninsulfonat von mindestens 80% und vorzugsweise mit niedrigem Gehalt an mehrwertigen Kationen. Der Sulfonierungsgrad kann in weiten Grenzen variieren.

Nichtionische Dispergiermittel oder Emulgatoren sind z.B. Umsetzungsprodukte von Alkylenoxiden mit alkylierbaren Verbindungen, wie z.B. Fettalkoholen, Fettaminen, Fettsäuren, Phenolen, Alkylphenolen, Arylalkylphenolen und Carbonsäureamiden.

Hierbei handelt es sich z.B. um Ethylenoxid-Addukte aus der Klasse der Umsetzungsprodukte von Ethylenoxid mit:
a) gesättigten und/oder ungesättigten Fettalkoholen mit 6 bis 20 C-Atomen; oder
b) Alkylphenolen mit 4 bis 12 C-Atomen im Alkylrest, oder
c) gesättigten und/oder ungesättigten Fettaminen mit 14 bis 20 C-Atomen, oder
d) gesättigten und/oder ungesättigten Fettsäuren mit 14 bis 20 C-Atomen.

Als Ethylenoxid-Addukte sind im einzelnen genannt:
a) Umsetzungsprodukte von gesättigten und/oder ungesättigten Fettalkoholen mit 6 bis 20 C-Atomen, mit 5 bis 30 Mol Ethylenoxid
b) Umsetzungsprodukte von Alkylphenolen mit 4 bis 12 C-Atomen mit 5 bis 20 Mol Ethylenoxid
c) Umsetzungsprodukte von gesättigten und/oder ungesättigten Fettaminen mit 14 bis 20 C-Atomen mit 5 bis 20 Mol Ethylenoxid
d) Umsetzungsprodukte von gesättigten und/oder ungesättigten Fettsäuren mit 14 bis 20 C-Atomen mit 5 bis 20 Mol Ethylenoxid.

Weitere bevorzugte Dispergiermittel sind alkoxylierte Styrol-Phenol-Kondensationsprodukte, die gegebenenfalls im Gemisch mit ihren anorganichen Estern, die durch Umsetzung des alkoxylierten Styrol-Phenol-Kondesationsproduktes mit anorganischen Säuren, wie beispielsweise Amidosulfonsäure, erhalten werden, eingesetzt werden.

Insbesondere zum Färben von Polyester eigenen sich auch Mischungen von Farbstoffen der Formel (I), wodurch unter Umständen das Zieh- und Aufbauvermögen der Farbstoffe sowie ihre Dispergierbarkeit verbessert werden können.

Die neuen Farbstoffmischungen können nach verschiedenen Verfahren hergestellt werden:
1. durch Abmischen der separat hergestellten und formierten Einzelfarbstoffkomponenten,
2. durch gemeinsame Formierung der separat hergestellten Einzelkomponenten,
3. durch gemeinsame Synthese von Mischungen der Farbstoffe der Formel (I) aus Mischungen unterschiedlicher Vorprodukte.

Die Mischung der Farbstoffe erfolgt zweckmäßigerweise in geeigneten Mühlen, beispielsweise Kugel- oder Sandmühlen. Getrennt formierte Einzelfarbstoffe können aber auch durch Einrühren in Färbeflotten gemischt werden.

Besonders geigenet sind Mischungen von Farbstoffen der Formel (I), die sich lediglich in dem Rest R₁ unterscheiden.

Die Farbstoffe eignen sich jedoch auch hervorragend zur Herstellung von Mischungen mit anderen Dispersionsfarbstoffen zur Erzeugung z.B. von Braun-, Grau- oder Grüntönen auf der Faser, weil sie die Lichtechtheit dieser Farbstoffe nicht beeinträchtigen.

Eine weitere bevorzugte Ausführungsform der vorliegenden Erfindung betrifft Mischungen von einem oder mehreren der Farbstoffe der Formel (I) mit einem oder mehreren Farbstoffen, wie sie üblicherweise zum Färben von Polyesterfasern oder Polyestertextilmaterialien für Automobilbezugsstoffe verwendet werden. Bei diesen Farbstoffen zum Färben von Automobilbezugsstoffen kann es sich insbesondere um Azo- Disazo-, Anthrachinon-, Nitro-, Naphthalimid- und Terephthalimid-Farbstoffe handeln. Besonders bevorzugte Farbstoffe für derartige Mischungen sind z.B. die Colour-Index-Farbstoffe Yellow 23, 42, 51, 59, 65, 71, 86, 108, 122, 163, 182, 211, Orange 29, 30, 32, 41, 44, 45, 61, 73, Rot 60, 82, 86, 91, 92, 127, 134, 138, 159, 167, 191, 202, 258, 279, 284, 302, 323, Blau 27, 54, 56, 60, 73, 77, 79, 79:1, 87, 266, 333, 361 Violet 27, 28, 57, und 95, wobei sich die Gewichtsverhältnisse der Farbstoffmischungen nach der gewünschten Farbnuance richten.

### Beispiele:

### Beispiel 1:

86,1 g (0,55 Mol) technisches Aminoiminoisoindolenin (92,8 %-ig), 67 g 2-Aminobenzoxazol und 5 g Piperazin wurden mit 500 ml Methanol verrührt und die Mischung 5 Stunden unter Rückfluß erhitzt. Danach wurde der Ansatz auf Raumtemperatur abgekühlt und nach Zutropfen von 200 ml Wasser das ausgefallene Produkt abgesaugt und mit Wasser gewaschen. Nach dem Trocknen erhielt man 87,9 g eines Produktes der Formel:

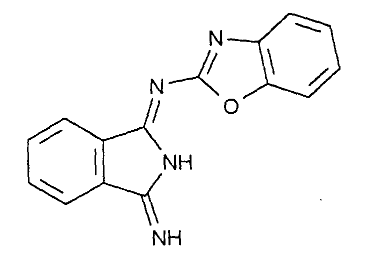

Das Produkt wurde durch Umkristallisieren aus DMF weiter gereinigt.

| Elementaranalyse: C₁₅H₁₀N₄O; MG = 262,3 g/mol | | | |
|---|---|---|---|
| | C | H | N |
| berechnet | 68,7 | 3,8 | 21,4 |
| gefunden | 68,9 | 3,6 | 21,1 |

### Beispiel 2:

Zu einer Mischung aus 80 ml Methanol und 80 ml einer 30%-igen Natriummethylatlösung wurden in 30 Minuten 54,9 g Phthalsäuredinitril gegeben und dabei die Temperatur durch leichtes Kühlen bei 20 - 25°C gehalten. Danach wurden in ca. 15 Minuten 53,6 g 2-Aminobenzoxazol eingestreut und der Ansatz noch 5 Stunden bei Raumtemperatur nachgerührt. Anschließend erhitzte man noch 15 Minuten auf 40°C, tropft 25 ml Eisessig zu und hält weitere 30 Minuten bei 60°C. Das ausgefallenen Produkt wurde bei Raumtemperatur abgesaugt und mit Wasser und Methanol gewaschen. Man erhielt 84,8 g eines Produktes der gleichen Formel wie bei Beispiel 1.

### Beispiel 3:

26,2 g (0,1 Mol) der Substanz von Beispiel 2 und 300 ml Amylalkohol wurden verrührt und 17 g (0,11 Mol) Cyanessigsäureamylester sowie 6 ml Eisessig zugegeben. Der Ansatz wurde 3 Stunden auf 80°C und danach noch kurz auf 100°C erhitzt. Nach Abkühlen auf Raumtemperatur wurde die ausgefallene Substanz abgesaugt und mit eiskaltem Methanol und mit Wasser gewaschen. Nach dem Trocknen erhielt man 29,2 g eines Produktes der folgenden Formel.

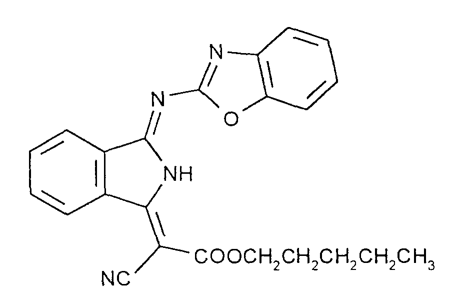

Der Farbstoff färbt Polyesterfasern in grünstichig gelben Tönen mit hervorragender Lichtechtheit.

### Beispiel 4:

26,2 g (0,1 Mol) der Substanz von Beispiel 2, 150 ml Wasser und 2,4 g Lignisol® SD 60, einem Dispergiermittel auf Basis von Ligninsulfonaten der Firma Borregaard, wurden verrührt, 6 ml Eisessig zugegeben und auf 70°C aufgeheizt. Danach wurden in 30 Minuten 17,1 g Cyanessigsäureamylester zugetropft, auf 90°C erhitzt und die Temperatur 5 Stunden lang gehalten. Das ausgefallene Produkt wurde bei Raumtemperatur abgesaugt und mit Wasser und Methanol gewaschen. Man erhielt 36,5 g des gleichen Farbstoffes wie bei Beispiel 3.

### Beispiel 5:

Verfuhr man wie bei Beispiel 4, setzte jedoch an Stelle von Cyanessigsäureamylester Cyanessigsäurebutylglykolester ein, so erhielt man in vergleichbarer Ausbeute einen Farbstoff der Formel:

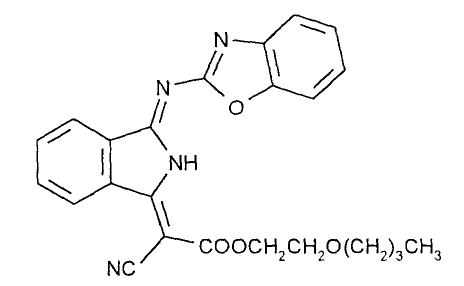

Der Farbstoff besitzt ähnliche Eigenschaften wie der Farbstoff von Beispiel 4, zeigt jedoch eine noch etwas bessere Lichtechtheit und eine noch bessere Sublimierechtheit.

Verfuhr man wie in den Beispielen 1 - 5 und setzte dabei in analoger Weise die entsprechenden Cyanessigsäureester und 2-Amino-benzoxazole ein, so erhielt man die in der folgenden Tabelle laufgeführten Farbstoffe

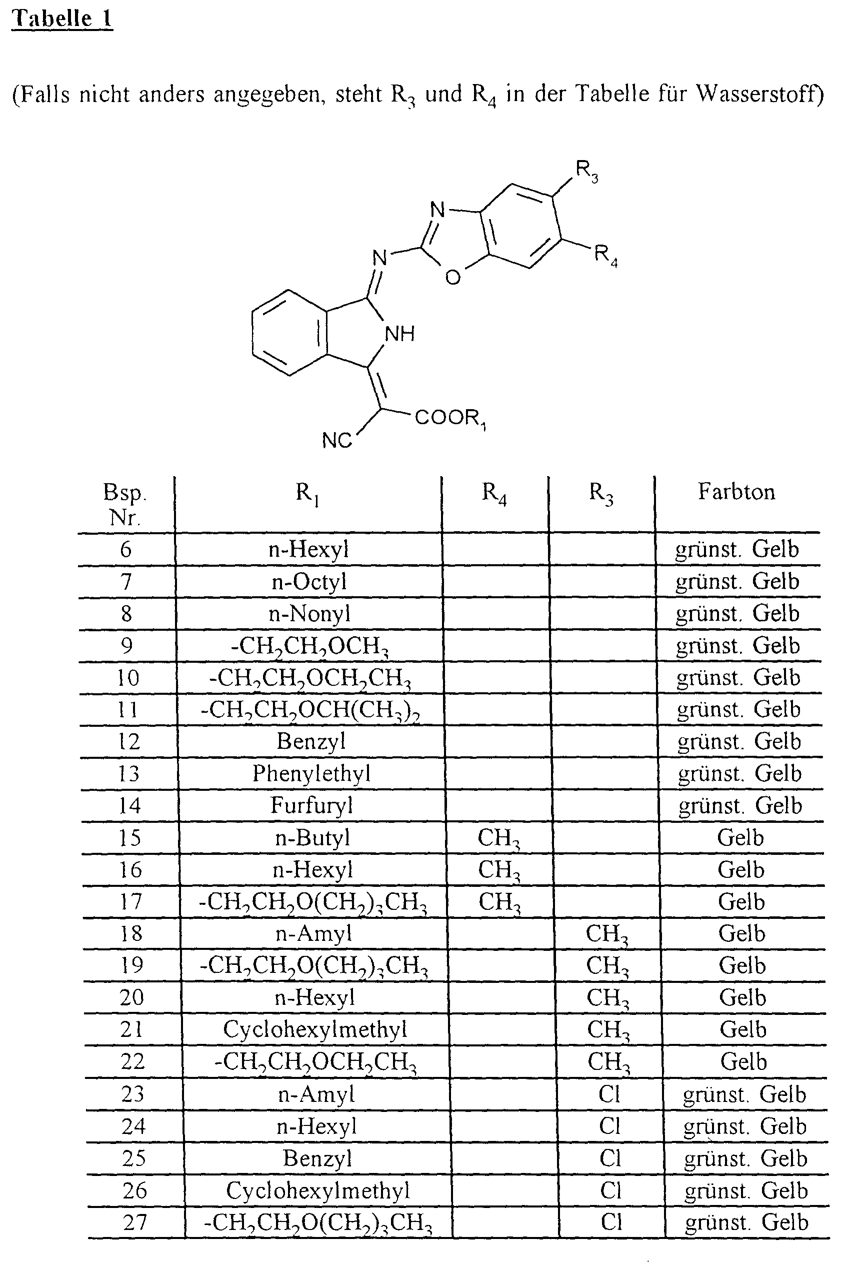

### Formierungsbeispiel 1

26 g des in Beispiel 5 erhaltenen Farbstoffs (in Form des wasserfeuchten Preßkuchens) wurden in 200 ml Wasser mit 55 g Ligninsulfonat, Na-Salz und 5 g eines nichtionischen Dispergiermittels (Additionsprodukt aus Abietinsäure und 50 Moläquivalente Ethylenoxid) versetzt und mit Schwefelsäure auf pH-Wert 7 gestellt. Anschließend wurde 1 Stunde bei Raumtemperatur in der Perlmühle bis zur Feinverteilung (90 % ≤ µm) gemahlen, gesiebt und im Sprühtrockner getrocknet.

### Formierungsbeispiel 2

Es wurde wie in Formierungsbeispiel 1 verfahren, jedoch ersetzte man die 5 g nichtionisches Dispergiermittel durch 5 g eines Tensidgemisches auf Basis eines alkoxylierten Styrol-Phenol-Kondensationsproduktes (Phenol/Styrol = 2,8:1, 29 Moläquivalente Ethylenoxid) und deren anorganischen Ester (Amidosulfonsäure), das zusätzlich ein Kondensationsprodukt aus Ölsäure und 6,5 Moläquivalente Ethylenoxid enthält.

### Anwendungsbeispiel 1

2 g des nach Formierungsbeispiel 1 erhaltenen Pulvers wurden in 1 000 g Wasser dispergiert. Die Dispersion wurde mit 0,5 bis 2 g/l eines handelsüblichen Dispergiermittels auf Basis eines Kondensationsproduktes aus Naphthalinsulfonsäurenatriumsalz und Formaldehyd, 0,5 bis 2 g/l Mononatriumphosphat und 2 g eines handelsüblichen Egalisierhilfsmittles versetzt und mit Essigsäure auf einen pH-Wert von 4,5 bis 5,5 gestellt. In die so erhaltene Färbeflotte brachte man 100 g eines texturierten Polyestergewebes auf Basis Polyethylenglykolterephthalat ein und färbt 60 Minuten bei 130°C.

### Anwendungsbeispiel 2

In analoger Weise zu Beispiel 1 wurde auch das nach Formierbeispiel 2 erhaltene Farbstoffpräparat zum Färben verwendet.

Man erhielt farbstarke brillante Gelbfärbungen mit hervorragenden coloristischen Eigenschaften wie Lichtechtheit, wobei die thermische Stabilität der Farbstoff dispersion und die Egalität der Färbung aus Anwendungsbeispiel 2 noch verbessert ist.

## Patentansprüche

1. Verbindungen, die der Formel (I) oder deren tautomeren Formen entsprechen 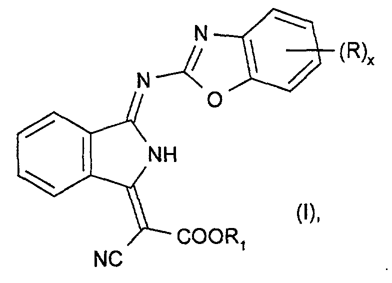 worin
R₁ für einen gesättigten oder ungesättigten, durch CN, gesättigten oder ungesättigten Oxyrest mit 1-4 C-Atomen substituierten oder unsubstituierten aliphatischen Rest mit 1 bis 12 C-Atomen, der gegebenenfalls durch ein oder mehrere Sauerstoffatome unterbrochen ist, einen unsubstituierten oder durch CN, gesättigten oder ungesättigten Oxyrest mit 1 bis 4 C-Atomen substituierten cycloaliphatischen Rest mit 5 bis 12 C-Atomen oder für einen unsubstituierten oder durch CN, gesättigten oder ungesättigten Oxyrest mit 1 bis 4 C-Atomen substituierten araliphatischen Rest mit 7 bis 20 C-Atomen steht,
x eine Zahl von 0 bis 4, vorzugsweise 0, 1 oder 2, bedeutet,
R gleich oder verschieden ist und für Halogen, C₁-C₁₀-Alkyl, gesättigtes oder ungesättigtes C₁-C₁₀-Alkoxy oder -Alkoxyalkoxy, CN, NO₂ oder, sofern x größer als 1, für den Rest eines anellierten Benzolringes steht.

2. Verbindungen gemäß Anspruch 1, worin
R gleich oder verschieden ist und für Cl, Br, C₁-C₄-Alkyl, gesättigtes oder ungesättigtes C₁-C₄-Alkoxy oder -Alkoxyalkoxy, CN, NO₂ oder, sofern x größer als 1, für den Rest eines anellierten Benzolringes steht.

3. Verbindungen gemäß Anspruch 1, worin
R₁ für einen geradkettigen aliphatischen Rest mit 4 bis 8 C-Atomen steht, der gegebenenfalls durch ein Sauerstoffatom unterbrochen ist und
R für Cl, Methyl, Methoxy oder Ethoxy steht und
x für 0 oder 1 steht.

4. Verbindungen gemäß Anspruch 1, worin
x 0 bedeutet.

5. Verfahren zur Herstellung von Verbindungen gemäß Anspruch 1, **dadurch gekennzeichnet, daß** man entweder eine Verbindung der Formel (II) 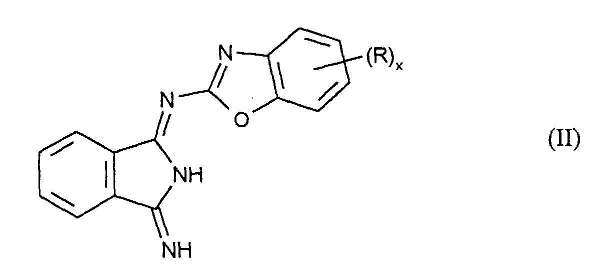 mit einem Cyanessigester der Formel (III)
NCCH₂COOR₁ (III)
oder
eine Verbindung der Formel (IV) 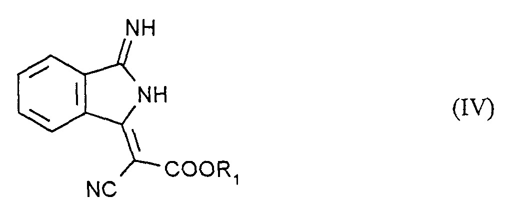 mit 2-Aminobenzoxazol der Formel (V) 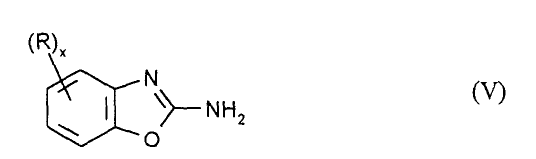 kondensiert, wobei
R, R₁ und x die in Anspruch 1 angegebene Bedeutung besitzen.

6. Verbindungen der Formel (II) 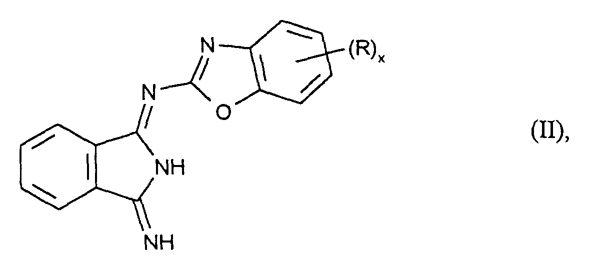 worin
x eine Zahl von 0 bis 4, vorzugsweise 0, 1 oder 2 bedeutet, und
R gleich oder verschieden ist und für Halogen, C₁-C₁₀-Alkyl, gesättigtes oder ungesättigtes C₁-C₁₀-Alkoxy oder -Alkoxyalkoxy, CN, NO₂ oder, sofern x größer als 1, für den Rest eines anellierten Benzolringes steht.

7. Verfahren zur Herstellung von Verbindungen gemäß Anspruch 6, **dadurch gekennzeichnet, daß** man Aminoiminoisoindolenin der Formel (VI) 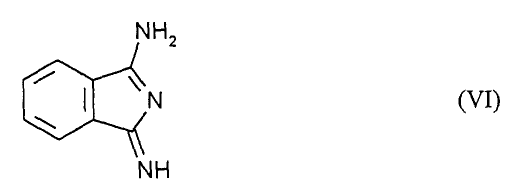 mit 2-Aminobenzoxazolen der Formel (V) 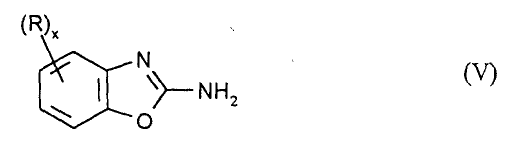 umsetzt, worin
R und x die in Anspruch 6 genannten Bedeutungen haben.

8. Verfahren zur Herstellung von Verbindungen gemäß Anspruch 6, **dadurch gekennzeichnet, daß** man Verbindungen der Formel (VIIa) und/oder (VIIb) 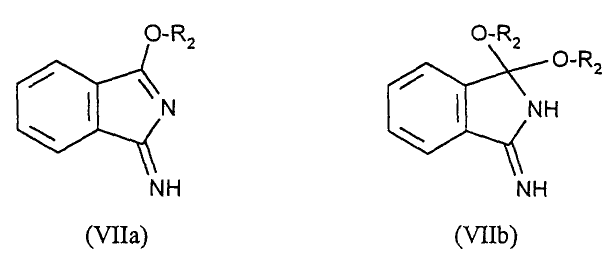 mit Verbindungen der Formel (V) 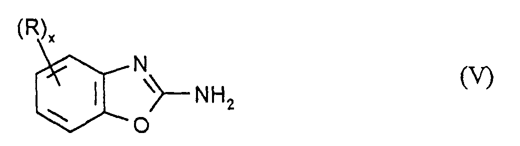 umsetzt, wobei
R und x die in Anspruch 6 angegebene Bedeutung besitzen und
R² für einen aliphatischen Rest, insbesondere für C₁-C₄-Alkyl steht.

9. Verwendung von Verbindungen gemäß Anspruch 1 zum Färben und Bedrucken von vollsynthetischen oder halbsynthetischen hochmolekularen Stoffen.

## Claims

1. Compounds which correspond to the formula (I) or tautomeric forms thereof 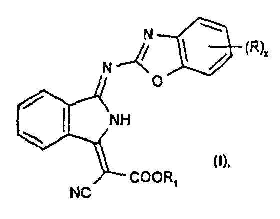 wherein
R₁ represents a saturated or unsaturated aliphatic radical having 1 to 12 C atoms, which is unsubstituted or substituted by CN or a saturated or unsaturated oxy radical having 1 to 4 C atoms and which is optionally interrupted by one or more oxygen atoms, a cycloaliphatic radical having 5 to 12 C atoms which is unsubstituted or substituted by CN or a saturated or unsaturated oxy radical having 1 to 4 C atoms or an araliphatic radical having 7 to 20 C atoms which is unsubstituted or substituted by CN or a saturated or unsaturated oxy radical having 1 to 4 C atoms,
x denotes a number from 0 to 4, preferably 0, 1 or 2, and
R is identical or different and represents halogen, C₁-C₁₀-alkyl, saturated or unsaturated C₁-C₁₀-alkoxy or -alkoxyalkoxy, CN, NO₂ or, if x is greater than 1, the radical of a fused-on benzene ring.

2. Compounds according to Claim 1, wherein
R is identical or different and represents Cl, Br, C₁-C₄-alkyl, saturated or unsaturated C₁-C₄-alkoxy or -alkoxyalkoxy, CN, NO₂ or, if x is greater than 1, the radical of a fused-on benzene ring.

3. Compounds according to Claim 1, wherein
R₁ represents a straight-chain aliphatic radical having 4 to 8 C atoms, which is optionally interrupted by an oxygen atom, and
R represents Cl, methyl, methoxy or ethoxy and
x represents 0 or 1.

4. Compounds according to Claim 1, wherein
x denotes 0.

5. Process for the preparation of compounds according to Claim 1, **characterized in that** either a compound of the formula (II) 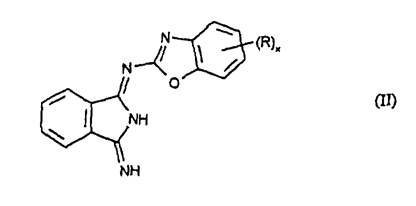 is subjected to a condensation reaction with a cyanoacetic ester of the formula (III)
NCCH₂COOR₁ (III)
or
a compound of the formula (IV) 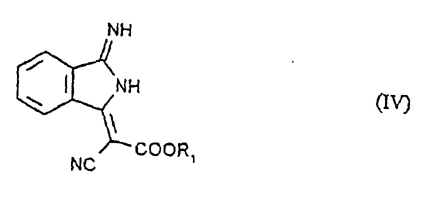 is subjected to a condensation reaction with 2-aminobenzoxazole of the formula (V) 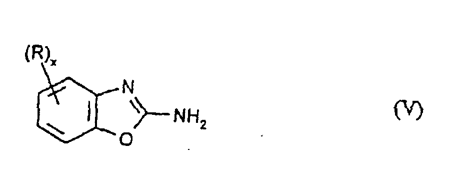 wherein
R, R₁ and x have the meaning given in Claim 1.

6. Compounds of the formula (II) 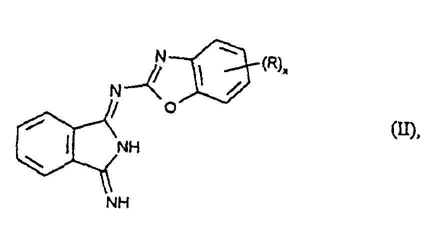 wherein
x denotes a number from 0 to 4, preferably 0, 1 or 2, and
R is identical or different and represents halogen, C₁-C₁₀-alkyl, saturated or unsaturated C₁-C₁₀-alkoxy or -alkoxyalkoxy, CN, NO₂ or, if x is greater than 1, the radical of a fused-on benzene ring.

7. Process for the preparation of compounds according to Claim 6, **characterized in that** aminoiminoisoindolenine of the formula (VI) 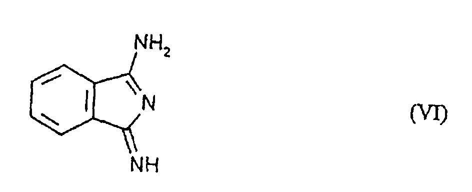 is reacted with 2-aminobenzoxazoles of the formula (V) 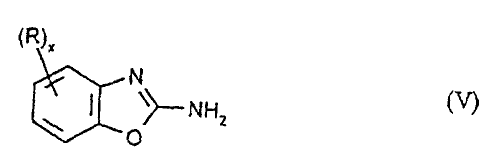 wherein
R and x have the meanings given in Claim 6.

8. Process for the preparation of compounds according to Claim 6, **characterized in that** compounds of the formula (VIIa) and/or (VIIb) 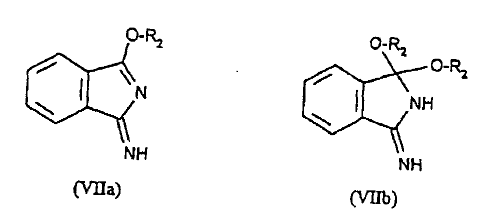 are reacted with compounds of the formula (V) 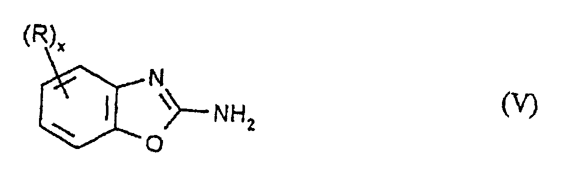 wherein
R and x have the meaning given in Claim 6 and
R₂ represents an aliphatic radical, in particular C₁-C₄-alkyl.

9. Use of compounds according to Claim 1 for dyeing and printing fully synthetic or semisynthetic high molecular weight substances.

## Revendications

1. Composés qui correspondent à la formule (I) ou ses formes tautomères 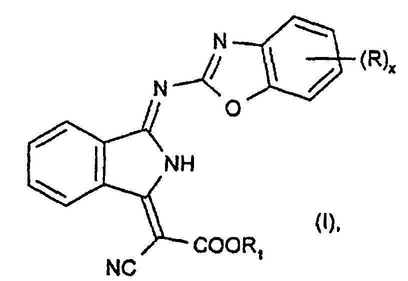 formule dans laquelle
R₁ représente un radical aliphatique saturé ou insaturé ayant de 1 à 12 atomes de carbone, non substitué ou substitué par CN ou par un radical oxy saturé ou insaturé ayant de 1 à 4 atomes de carbone, qui est éventuellement interrompu par un ou plusieurs atomes d'oxygène, un radical cycloaliphatique ayant de 5 à 12 atomes de carbone, non substitué ou substitué par CN ou par un radical oxy saturé ou insaturé ayant de 1 à 4 atomes de carbone, ou un radical araliphatique ayant de 7 à 20 atomes de carbone, non substitué ou substitué par CN ou par un radical oxy saturé ou insaturé ayant de 1 à 4 atomes de carbone,
x représente un nombre allant de 0 à 4, de préférence 0, 1 ou 2, et
les radicaux R sont identiques ou différents et représentent un atome d'halogène, un groupe alkyle en C₁-C₁₀, alcoxy en C₁-C₁₀ ou alcoxy(C₁-C₁₀)alcoxy saturé ou insaturé, CN, NO₂ ou, si x est supérieur à 1, le reste d'un cycle benzénique soudé.

2. Composés selon la revendication 1, dans lesquels
les radicaux R sont identiques ou différents et représentent Cl, Br, un groupe alkyle en C₁-C₄, un groupe alcoxy en C₁-C₄ ou alcoxy(C₁-C₄)alcoxy saturé ou insaturé, CN, NO₂ ou, si x est supérieur à 1, le reste d'un cycle benzénique soudé.

3. Composés selon la revendication 1, dans lesquels
R₁ représente un radical aliphatique à chaîne droite ayant de 4 à 8 atomes de carbone, qui est éventuellement interrompu par un atome d'oxygène et
R représente Cl, le groupe méthyle, méthoxy ou éthoxy et
x représente 0 ou 1.

4. Composés selon la revendication 1, dans lesquels
x représente 0.

5. Procédé pour la préparation des composés selon la revendication 1, **caractérisé en ce qu'**on condense soit un composé de formule (II) 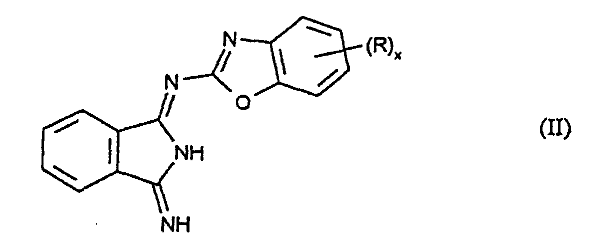 avec un ester d'acide cyanoacétique de formule (III)
NCCH₂COOR₁ (III)
soit
un composé de formule (IV) 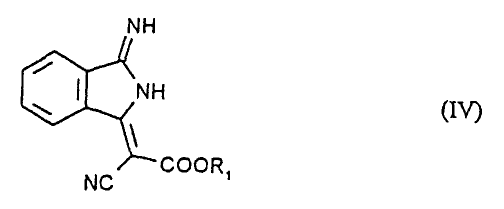 avec un 2-aminobenzoxazole de formule (V) 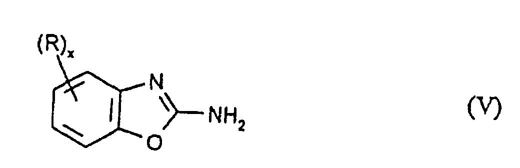 dans lesquelles R, R₁ et x ont les significations données dans la revendication 1.

6. Composés de formule (II) 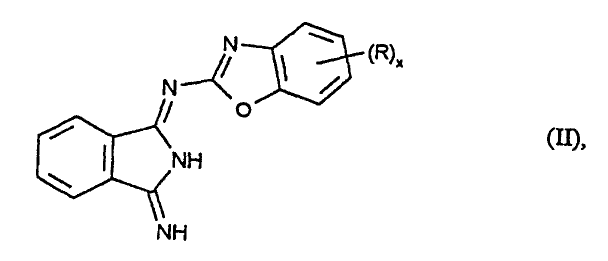 dans laquelle
x représente un nombre allant de 0 à 4, de préférence 0, 1 ou 2, et
les radicaux R sont identiques ou différents et représentent un atome d'halogène, un groupe alkyle en C₁-C₁₀, un groupe alcoxy en C₁-C₁₀ ou alcoxy(C₁-C₁₀)alcoxy saturé ou insaturé, CN, NO₂ ou, si x est supérieur à 1, le reste d'un cycle benzénique soudé.

7. Procédé pour la préparation des composés selon la revendication 6, **caractérisé en ce qu'**on fait réagir l'amino-imino-iso-indolénine de formule (VI) 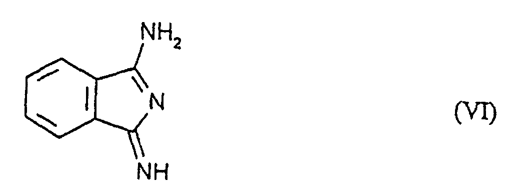 avec des 2-aminobenzoxazoles de formule (V) 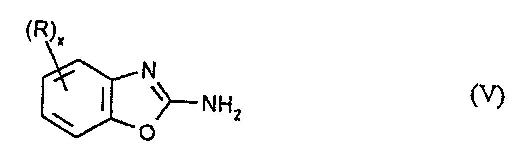 dans laquelle
R et x ont les significations données dans la revendication 6.

8. Procédé pour la préparation des composés selon la revendication 6, **caractérisé en ce qu'**on fait réagir des composés de formule (VIIa) et/ou (VIIb) 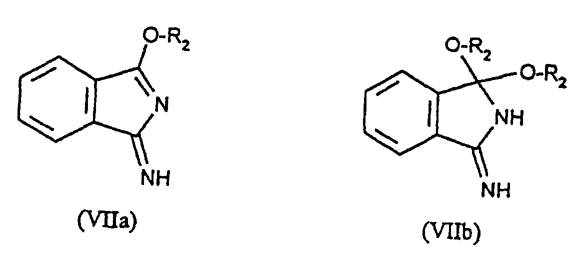 avec des composés de formule (V) 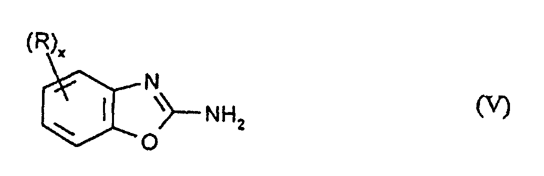 R et x ayant les significations données dans la revendication 6 et
R₂ représentant un radical aliphatique, en particulier un groupe alkyle en C₁-C₄.

9. Utilisation des composés selon la revendication 1, pour la teinture et l'impression de matières entièrement synthétiques ou semi-synthétiques de masse moléculaire élevée.
